Europäisches Patentamt

European Patent Office

Office européen des brevets

(19)

(11) Veröffentlichungsnummer: **0 218 007**
**B1**

(12) **EUROPÄISCHE PATENTSCHRIFT**

(45) Veröffentlichungstag der Patentschrift:
**31.01.90**

(21) Anmeldenummer: **86107946.5**

(22) Anmeldetag: **11.06.86**

(51) Int. Cl.⁴: **A61N 1/36**

(54) **Herzschrittmacher.**

(30) Priorität: **04.10.85 DE 3535534**

(43) Veröffentlichungstag der Anmeldung:
**15.04.87 Patentblatt 87/16**

(45) Bekanntmachung des Hinweises auf die Patenterteilung:
**31.01.90 Patentblatt 90/5**

(84) Benannte Vertragsstaaten:
**DE FR GB IT NL SE**

(56) Entgegenhaltungen:
**EP-A- 0 009 255**
**EP-A- 0 089 014**
**GB-A- 2 070 282**
**US-A- 3 523 539**
**US-A- 3 593 718**

(73) Patentinhaber: **Siemens Aktiengesellschaft,
Wittelsbacherplatz 2, D-8000 München 2(DE)**
(84) Benannte Vertragsstaaten: **DE FR GB IT NL**

(73) Patentinhaber: **Siemens Elema AB, Röntgenvägen 2,
S-171 95 Solna 1(SE)**
(84) Benannte Vertragsstaaten: **SE**

(72) Erfinder: **Lekholm, Anders, Dr., Gnejsvägen 4,
S-161 39 Bromma(SE)**

ACTORUM AG

## Beschreibung

Während der Atmung eines Patienten ändert sich die elektrische Impedanz zwischen zwei Elektroden auf der Brust. Durch Erfassung der Impedanz ist es deshalb möglich, die Atmungsrate und die Tiefe der Atmung zu berechnen. Daraus kann wiederum auch das ventilierte Atemminutenvolumen bestimmt werden. Das Impedanzsignal kann auch zur Bestimmung der Stimulationsrate eines Herzschrittmachers verwendet werden, wie in US-A 3 593 718 beschrieben wird.

Der Erfindung liegt die Aufgabe zugrunde, einen Herzschrittmacher so auszubilden, daß mit ihm von der Atmung des Patienten abhängige elektrische Signale gebildet werden, die zur physiologischen Steuerung herangezogen werden.

Diese Aufgabe ist erfindungsgemäß gelöst durch Mittel zur Bildung von dem Stimulationsstrom und der Stimulationsspannung entsprechenden elektrischen Signalen und einen Dividierer für diese Signale zur Bildung eines der Elektrodenimpedanz entsprechenden Impedanzsignales, welches nach Herausfilterung des von der Atmung abhängigen Signalanteiles einer Steuerstufe für den Pulsgenerator zugeführt ist. Teile des Impedanzsignales hängen in diesem Fall von der Atmung des Patienten ab und können zur Steuerung des Pulsgenerators des Herzschrittmachers herangezogen werden. Bei der vorliegenden Erfindung wird das Atemsignal entgegen dem Stand der Technik aus den Stimulationsimpulsen über die Standardelektrode, also ohne zusätzliche Meßelektroden und ohne zusätzliche Meßenergie und Energie zur Erzeugung von Meßsignalen, gewonnen.

Normale Atmungsraten liegen in Ruhe zwischen 10 und 20 Atemzyklen pro Minute. Da die normale Herzschrittmacher-Stimulationsrate etwa 70 Stimulationsimpulse pro Minute beträgt, können ungefähr fünf Meßvorgänge für die Elektrodenimpedanz pro Atmungszyklus durchgeführt werden. Dies ist völlig ausreichend, um die Atemrate und das Atemminutenvolumen zu erfassen.

Bei einem Herzschrittmacher, bei dem die Stimulationsrate veränderlich ist, kann das genannte Verhältnis bei Verstärkung der Atmung (Zunahme der Atemfrequenz) durch eine Erhöhung der Stimulationsrate beibehalten werden. Es kann auch eine variierende Abtastrate für die Messung der Atmungsparameter angewandt werden.

Die Erfindung ist nachfolgend anhand der Zeichnungen näher erläutert. Es zeigen:

Fig. 1 und 2 zwei Ausführungsbeispiele zur Gewinnung und Weiterverarbeitung von Atemsignalen bei einem Herzschrittmacher nach der Erfindung, und

Fig. 3 und 4 Kurven zur Erläuterung der Figuren 1 und 2.

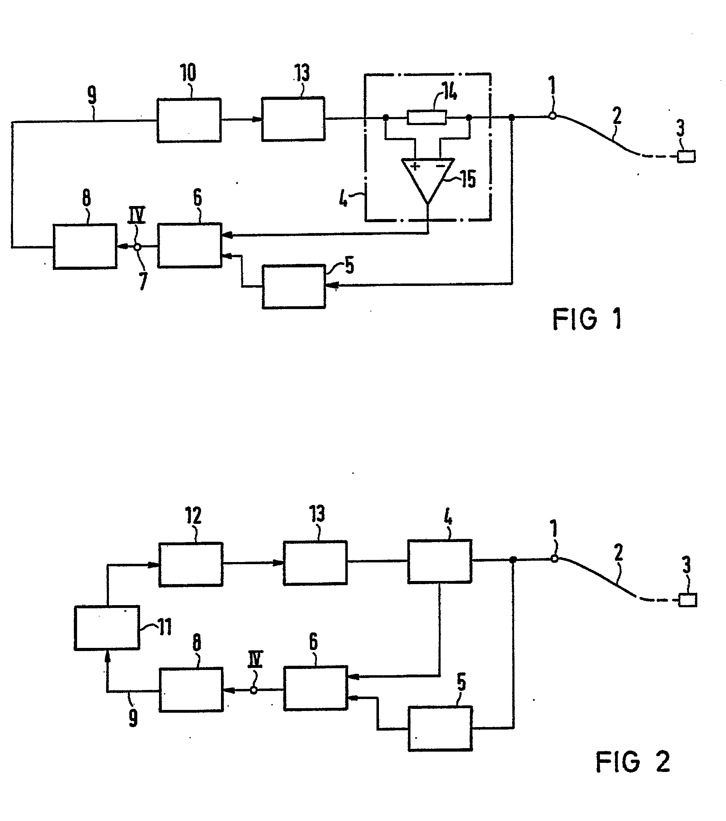

In der Figur 1 ist eine Klemme 1 dargestellt, von der eine Leitung 2 zu einer schematisch dargestellten Herzschrittmacherelektrode 3 führt. Der Pulsgenerator des Herzschrittmachers ist mit 13 bezeichnet. Die Stimulationsimpulse durchfließen einen Widerstand 14, der zusammen mit einem Verstärker 15 ein Strommeßglied 4 bildet. Die Spannung der Stimulationsimpulse wird durch ein Spannungsmeßglied 5 gemessen. Demgemäß werden den beiden Eingängen eines Dividierers 6 dem Stimulationsstrom und der Stimulationsspannung entsprechende Signale zugeführt, der daraus durch Division ein am Ausgang 7 liegendes, der Elektrodenimpedanz entsprechendes elektrisches Signal bildet. Dieses Signal wird über ein Filter 8, das die von der Atmung abhängigen Signalanteile herausfiltert, in ein der Atemrate entsprechendes Signal auf der Leitung 9 umgewandelt, das über eine Steuerstufe 10 die Steuerung des Pulsgenerators 13 in Abhängigkeit von der Atemrate bewirkt.

In der Figur 2 sind Teile, die mit Teilen der Figur 1 gleich sind, mit gleichen Bezugzeichen bezeichnet. Bei diesem Ausführungsbeispiel wird das der Atemrate entsprechende Signal auf der Leitung 9 einem Integrator 11 zugeführt, der daraus ein dem Atemminutenvolumen entsprechendes Signal bildet, das über eine Steuerstufe 12 wieder die Impulsrate des Pulsgenerators 13 in Abhängigkeit vom Atemminutenvolumen beeinflußt.

Wesentlich für die beiden Beispiele gemäß den Figuren 1 und 2 ist demgemäß, daß über die Elektrodenimpedanz des Herzschrittmachers die Atmung des Patienten erfaßt und ein Atemratensignal bzw. ein Atemminutenvoluem-Signal gebildet wird. Diese Signale steuern dann die Impulsrate des Pulsgenerators 13 des Herzschrittmachers.

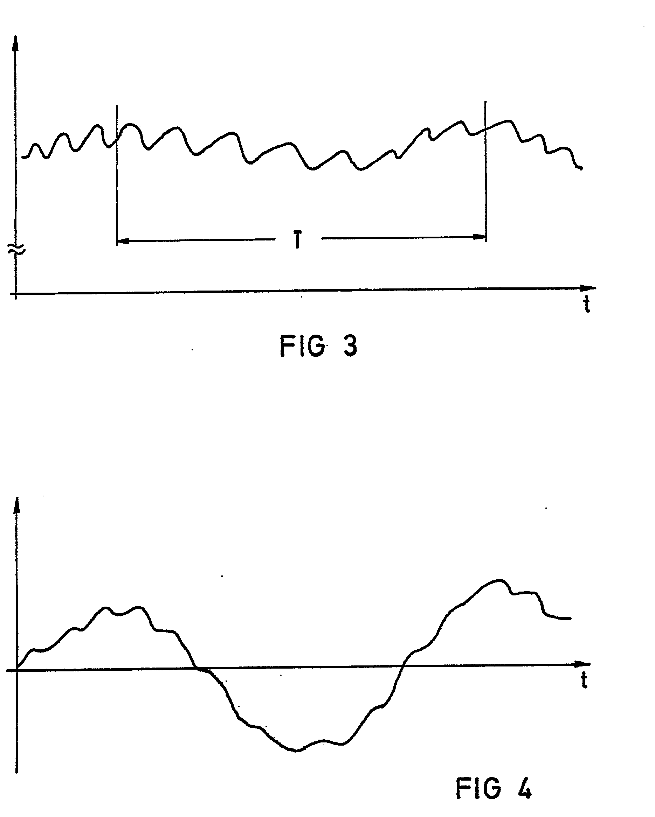

Die Figur 3 zeigt den zeitlichen Verlauf der Elektrodenimpedanz. In der Figur 3 ist ein Atemintervall T eingetragen. Die Elektrodenimpedanz schwankt zyklisch mit der Atmung.

Die Figur 4 zeigt das Signal an der Stelle IV in den Figuren 1 und 2. d.h., ein von der Atmung des Patienten abhängiges Signal, das über die Messung der Elektrodenimpedanz gewonnen ist.

## Patentansprüche

1. Herzschrittmacher, **gekennzeichnet durch** Mittel (4, 5) zur Bildung von dem Stimulationsstrom und der Stimulationsspannung entsprechenden elektrischen Signalen und einen Dividierer (6) für diese Signale zur Bildung eines der Elektrodenimpedanz entsprechenden Impedanzsignales, welches nach Herausfilterung des von der Atmung abhängigen Signalanteiles einer Steuerstufe (10, 12) für den Pulsgenerator (13) zugeführt ist.

## Claims

1. A heart pace maker, characterised by means (4, 5) for forming electrical signals corresponding to the stimulation current and the stimulation voltage and a divider (6) for these signals for forming an impedance signal corresponding to the electrode impedance, which signal is supplied to a control stage (10, 12) for the pulse generator (13) after the respiration-dependent signal component has been filtered out.

**Revendications**

1. Stimulateur cardiaque, caractérisé par des moyens (4, 5) pour former des signaux électriques correspondant au courant de stimulation et à la tension de stimulation, et un diviseur (6) servant à diviser ces signaux pour former un signal d'impédance, qui correspond à l'impédance des électrodes et qui est envoyé, après séparation par filtrage de la composante de signal dépendant de la respiration, à un étage (10, 12) servant à commander le générateur d'impulsions (13).

EP 0 218 007 B1

FIG 1

FIG 2

FIG 3

FIG 4